# EUROPEAN PATENT SPECIFICATION

(11) **EP 1 779 897 B1**
(45) Date of publication and mention of the grant of the patent: **01.12.2010**
(21) Application number: 06255523.0
(22) Date of filing: 26.10.2006
(51) Int. Cl.: A61Q 5/12, A61Q 19/00, A61K 8/04, A61K 8/81

(54) **Cosmetic product, nanoparticles for cosmetics, and powder for cosmetics**
Kosmetisches Produkt, Nanopartikel für die Kosmetik, sowie Puder für die Kosmetik
Produit cosmétique, des nanoparticules pour la cosmétique, ainsi que des poudres pour la cosmétique

(30) Priority: 26.10.2005 JP 2005311298
(43) Date of publication of application: 02.05.2007
(73) Proprietor: NOF CORPORATION, Tokyo 150-0013 (JP)
(72) Inventor: Fukui, Hiroki, Tsukuba-shi Ibaraki 300-2635 (JP); Sekine, Yoshimi, Tsukuba-shi Ibaraki 300-2635 (JP); Kayaba, Daisuke, Tsukuba-shi Ibaraki 300-2635 (JP); Kang, Eui-chul, Tsukuba-shi Ibaraki 300-2635 (JP); Ogura, Atsuhiko, Tsukuba-shi Ibaraki 300-2635 (JP); Shuto, Kenshiro, Tsukuba-shi Ibaraki 300-2635 (JP)
(74) Representative: Hallybone, Huw George

(56) References cited:
- EP-A- 1 652 838
- FR-A- 2 835 430
- JP-A- 2006 232 673
- US-A1- 2005 187 341

## Description

The present invention relates to cosmetic products that have a good texture and an excellent barrier function for skin and hair, as well as nanoparticles for cosmetics and powder for cosmetics that are used in the cosmetic product.

Skin is roughened by deterioration of skin barrier function due to dryness in winter time, overcleansing, or ageing. In such skin conditions, decreases in sebum, intercellular lipids, and natural moisturizing factors are observed. In view of this, preparations for external use, such as cosmetics and medicine, for maintaining strong skin barrier function have been developed, and applied for protection of hair as well.

Ceramide, which is an intercellular lipid, has a confirmed role in skin barrier function, and has been widely studied for blending in skin preparations for external use, such as cosmetics. However, general properties of ceramides, such as high melting points, high crystallinity, and low compatibility with other compounds, severely restrict the manner of their incorporation into preparations for external use, and thus external preparations that could fully exhibit the function of ceramides are hard to be obtained.

JP-2000-239151-A, JP-2001-122724-A, JP-2003-55129-A, and JP-2003-300842-A discuss methods for stably incorporating ceramides.

However, the discussed methods still leave the problem of limited blending recipes, and cosmetics have not yet been obtained which can contain ceramides at high concentrations, have a good texture, allow full expression of the function of ceramides, and have strong skin barrier function and hair protection effect.

JP-9-241144-A, JP-10-226674-A, and JP-2001-316384-A propose ceramide-like novel compounds, but with insufficient effect. Thus there is a strong demand for development of cosmetics containing a still novel ceramide-like substance.

It is an object of the present invention to provide a cosmetic product that allows full expression of the inherent functions of ceramide as an intercellular lipid, such as skin barrier function and hair protection effect, and has a good texture.

It is another object of the present invention to provide nanoparticles for cosmetics and powder for cosmetics containing a ceramide-like polymer, which may be used in the cosmetic product mentioned above, exhibit good skin barrier function and hair protection effect, and are easy to incorporate into various cosmetic materials, as well as to provide use of the ceramide-like polymer for the manufacture of a cosmetic product.

The present inventors have made intensive studies for achieving the above objects to find out that particular polymers exhibit the desired effects, which polymers are obtained by polymerization of a monomer material containing a monomer having both a glycerol group and a urethane bond in its molecular structure, and having a chemical structure similar to that of ceramide, and that such polymers may easily be mixed with other cosmetic materials, to thereby complete the present invention.

According to the present invention, there is provided a cosmetic product comprising:
a cosmetic material, and
a polymer obtained by polymerization of a monomer material comprising a glycerol (meth)acrylate monomer (referred to as GU monomer herein below) and a monomer having a long-chain alkyl group,
   wherein said glycerol (meth)acrylate monomer is represented by the formula (1): 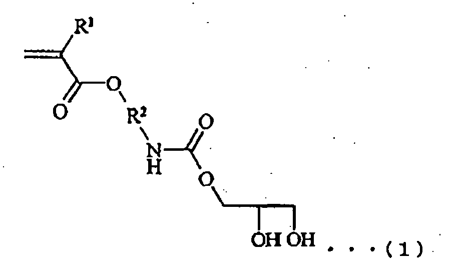 wherein R¹ stands for a hydrogen atom or a methyl group, and R² stands for -(CH₂)n- with n being an integer of 1 to 4, and
   said monomer having a long-chain alkyl group is represented by the formula (2): 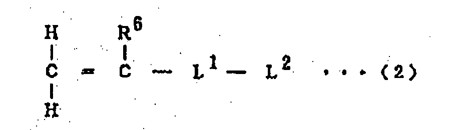 wherein L¹ stands for -C₆H₄-, -C₆H₁₀-, -(C=O) -O-, -O-, - (C=O) NH-, -O- (C=O) -, or -O- (C=O) -O-, L² stands for an alkyl group having 10 to 22 carbon atoms, and R⁶ stands for a hydrogen atom or a methyl group.

Polymer obtained by polymerization of a monomer material comprising GU monomer shall be referred to as GU polymer, herein below.

According to the present invention, there are provided nanoparticles for cosmetics comprising a GU polymer obtained by polymerization of a monomer material comprising a glycerol (meth)acrylate monomer represented by the formula (1), and having an average particle size of 5 to 500 nm.

According to the present invention, there is also provided powder for cosmetics comprising cosmetic material powder that has been surface-treated with a GU polymer obtained by polymerization of a monomer material comprising a glycerol (meth)acrylate monomer represented by the formula (1) and a monomer having a long-chain alkyl group represented by the formula (2).

According to the present invention, there is provided use of a GU polymer obtained by polymerization of a monomer material comprising a glycerol (meth)acrylate monomer represented by the formula (1) and a monomer having a long-chain alkyl group represented by the formula (2) , for the manufacture of a cosmetic product.

The present invention will now be explained in detail.

The cosmetic product according to the present invention contains a GU polymer obtained by polymerization of a monomer material containing a glycerol (meth)acrylate monomer represented by the formula (1) and a monomer having a long-chain alkyl group represented by the formula (2).

In the formula (1), R¹ stands for a hydrogen atom or a methyl group, with a methyl group being preferred for stability. R² stands for - (CH₂)n-, wherein n is an integer of 1 to 4. Specifically, R² is -CH₂-, -CH₂CH₂-, -CH₂CH₂CH₂-, or -CH₂CH₂CH₂CH₂-, with -CH₂CH₂- being preferred for availability.

Examples of the GU monomer may include glycerol-1-methacryloyloxyethyl urethane and glycerol-1-methacryloyloxypropyl urethane, with the former being preferred for its easiness of synthesis.

The GU monomer may be prepared, for example, by subjecting a cyclic ketal represented by the formula (3) and (meth) acryloyloxyalkyl isocyanate represented by the formula (4) to urethane reaction, and subjecting the resulting compound to hydrolytic ring-opening reaction in a water-containing solvent in the presence of a catalyst.

The urethane reaction may be carried out usually at 0 to 100°C for 6 to 24 hours. The hydrolytic ring-opening reaction may be carried out usually at 0 to 50 °C for about 1 to 6 hours in a water-containing solvent in the presence of a catalyst, such as an organic acid.

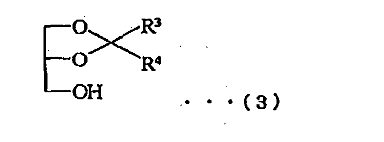

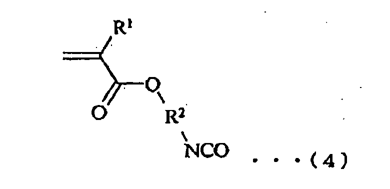

In the formula (3), R³ and R⁴ may either be the same or different, and each stands for a hydrogen atom, a methyl or ethyl group. In the formula (4), R¹ and R² are the same as those in the formula (1) above, and their preferred examples are as mentioned above.

The monomer material for preparing the GU polymer may either be the GU monomer alone or in mixture with other monomers that are copolymerizable with the GU monomer. Such other monomers may be selected from a wide variety of known polymerizable monomers, provided that they are copolymerizable with the GU monomer. For making the resulting GU polymer easy to form nanoparticles in cosmetics, a monomer having a long-chain alkyl group represented by the formula (2) (referred to as LA monomer) may be preferred: 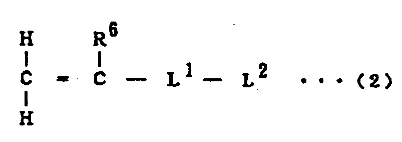 wherein L¹ stands for -C₆H₄-, -C₆H₁₀-, -(C=O)-O-, -O-, -(C=O)NH-, -O-(C=O)-, or -O-(C=O)-O-, L² stands for a straight or branched alkyl group having 10 to 22 carbon atoms, such as a decyl, dodecyl, tetradecyl, hexadecyl, octadecyl, or docosanyl group, and R⁶ stands for a hydrogen atom or a methyl group.

Examples of the LA monomer may include straight or branched alkyl(meth)acrylates, such as decyl(meth)acrylate, dodecyl(meth)acrylate, tetradecyl(meth)acrylate, hexadecyl(meth)acrylate, octadecyl(meth)acrylate, and docosanyl(meth)acrylate; and vinyl ester monomers, such as vinyl decanoate, vinyl dodecanoate,vinylhexadecanoate,vinyloctadecanoate, and vinyl docosanoate. For stability, for example, easiness of making the GU polymer nanoparticles, such as nanosphere, in the cosmetics, octadecylmethacrylate is particularly preferred. In the monomer material, the LA monomer may be a single monomer or a mixture of two or more kinds of monomers.

Examples of the monomers other than the LA monomer may include methyl(meth)acrylate, ethyl(meth)acrylate, butyl(meth)acrylate, 2-ethylhexyl(meth)acrylate, benzyl(meth)acrylate, phenoxyethyl(meth)acrylate, cyclohexyl(meth)acrylate, polypropylene glycol mono(meth)acrylate, polytetramethylene glycol mono(meth)acrylate, polypropylene glycol di(meth)acrylate, polytetramethylene glycol di(meth)acrylate, polypropylene glycol polyethylene glycol mono(meth)acrylate, glycidyl (meth)acrylate, (meth)acryloyloxypropyl trimethoxysilane, styrene, methylstyrene, chloromethylstyrene, methyl vinyl ether, butyl vinyl ether, vinyl acetate, vinyl propionate, 2-hydroxyethyl(meth)acrylate, 2-hydroxybutyl(meth)acrylate, 4-hydroxybutyl(meth)acrylate, (meth)acrylic acid, styrene sulfonic acid, (meth)acrylamide, (meth)acryloyloxy phosphonic acid, aminoethylmethacrylate, dimethylaminoethyl(meth)acrylate, 2-hydroxy-3-(meth)acryloxypropyl trimethylammonium chloride, and polyethylene glycol mono(meth)acrylate.

In the monomer material, the content of the GU monomer is 20 to 100 mol%, preferably 30 to 90 mol%. The content of the other monomers than the GU monomer, such as the LA monomer, if any, may be 10 to 80 mol%, preferably 20 to 70 mol%. In the monomer material, if the content of the GU monomer is less than 20 mol%, desired skin barrier function or hair protection effect may be hard to obtain. If the content of the other monomers, when contained, is less than 10 mol%, the effect of such other monomers may not be obtained.

When the GU polymer is to be made into nanoparticles to be discussed later, the monomer material preferably contains the GU monomer and the LA monomer. Here, the content of the GU monomer may preferably be 20 to 90 mol%, more preferably 15 to 60 mol% of the monomer material, and the content of the LA monomer may preferably be 10 to 80 mol%, more preferably 40 to 85 mol% of the monomer material. If the GU monomer content is less than 20 mol%, the resulting polymer may not be made into a stable nanoparticle dispersion, such as a nanosphere dispersion, whereas at over 90 mol%, the polymer may not be formed into nanospheres.

The molecular weight of the GU polymer is preferably 5000 to 5000000, more preferably 100000 to 2000000 in weight average molecular weight. At less than 5000, sufficient skin barrier function or hair protection effect may not be exhibited, whereas at over 5000000, the polymer may be hard to be incorporated into cosmetics.

The GU polymer may be prepared, for example, by subjecting the monomer material containing the GU monomer to bulk polymerization, to solution polymerization by adding a solution, or to dispersion polymerization in a dispersed state.

The solvent used in the solution or dispersion polymerizationmaybe any solvent, such as methanol, ethanol, isopropanol, n-propanol, butanol, dimethylformamide, dimethylsulfoxide, dimethylacetamide, acetonitrile, or ethyl acetate, a mixture of water and at least one of these organic solvents, or a variety of other solvents.

The monomer material containing the GU monomer may be polymerized by radical polymerization.

The radical polymerization may be carried out using a radical polymerization initiator. Examples of the radical polymerization initiator may include organic peroxides, such as benzoyl peroxide, t-butylperoxy neodecanoate, and succinic peroxide; and azo compounds, such as 2,2'-azobisisobutyronitrile and 2,2'-azobisdimethylisobutyrate, with 2,2'-azobisisobutyronitrile being preferred for its polymerization property, availability, and easy removability in purification.

A preferred amount of the radical polymerization initiator is usually 0.1 to 5.0 parts by weight based on 100 parts by weight of the monomer material. The temperature and time of the polymerization may suitably be decided depending on the kind of the radical polymerization initiator, presence/absence or the kind of other monomers. For example, for radical polymerization of the GU monomer alone, 2,2'-azobisdimethylisobutyrate may be used as the radical polymerization initiator, and suitable temperature and time of the polymerization may preferably be 50 to 70 °C and 8 to 48 hours, respectively.

The GU polymer obtained by radical polymerization may be purified and dried by conventional methods, such as reprecipitation, membrane separation, solvent extraction, supercritical extraction, extractive distillation, freeze drying, and spray drying. The content of the impurities such as residual monomers or organic solvent may be made usually not more than 5000 ppm, preferably not more than 500 ppm.

The GU polymer, when made into nanoparticles, provides excellent emollient effect and allows encapsulation of lipophilic active components in the cosmetic product of the present invention. The nanoparticles for cosmetics obtained by making the GU polymer into nanoparticles are particularly useful for cosmetics for skin and external preparation for hair, among the cosmetic product of the present invention.

The nanoparticles of the GU polymer may be prepared, for example, by copolymerizing a monomer material containing the GU monomer and the LA monomer as mentioned above, followed by processing to be discussed below. Hereinbelow, the polymer obtained by copolymerization of a monomer material containing the GU monomer and the LA monomer is referred to as GU-LA polymer.

The average particle size of the nanoparticles in the cosmetic product is usually 5 to 500 nm, preferably 10 to 200 nm. At over 500 nm, the nanoparticles tend to aggregate to lower the stability when made into a dispersion, and may present a rough texture in the cosmetic product. The average particle size of the nanoparticles may be measured with a commercial measuring device which employs the dynamic light scattering as its principle of measurement.

The GU polymer may be made into nanoparticles by, for example, conventional emulsification, such as vacuum emulsification, high pressure emulsification, phase-inversion emulsification, gel emulsification, melt emulsification, multiphase emulsification, or forced mechanical emulsification. Alternatively, the nanoparticles may also be prepared by dissolving the GU polymer, such as the GU-LA polymer, in a highly polar solvent, such as alcohol or alcohol/water, and adding dropwise the resulting solution under stirring into water to spontaneously form the nanoparticles. In the latter method, when a lipophilic component is added to the alcohol or alcohol/water solution of the GU polymer, an oil-soluble components, which is usually hardly water soluble, may be encapsulated stably in the nanoparticles and dispersed in water. Such encapsulation improves the feel of use and stability of the encapsulated component.

Examples of the alcohol to be used for preparing the nanoparticles may include methanol, ethanol, n-propanol, i-propanol, n-butanol, i-butanol, t-butanol, ethylene glycol, propanediol, butanediol, and glycerin, with ethanol, n-propanol, i-propanol, glycerin, and butanediol being particularly preferred. Two or more of these may be used in mixture.

Examples of the component that may be encapsulated in the nanoparticles may include antioxidants, such as vitamin A, vitamin E, polyphenols, astaxanthin, and catechin; whitening agents, such as vitamin C derivatives, kojic acid, placenta extract, arbutin, ellagic acid, rucinol (4-n-butylresorcinol), and linoleic acid; oils and fats, such as squalane and olive oil; anti-ageing agents, such as chelating agent, N-methyl-L-serine, and ursolic acid; UV absorbers, such as paraaminobenzoic acid derivatives, cinnamic acid derivatives, benzophenone derivatives, and salicylic acid derivatives; UV reflectors, such as titanium oxide and zinc oxide; astringent agents, such as caffeine and organic iodine; various moisturizing agents; various flavoring agents; various antibacterial agents; and various disinfectants.

The powder for cosmetics is powder obtained by surface-treating cosmetic material powder with the GU polymer to at least partially coat the external surface of the cosmetic material powder with the GU polymer.

The cosmetic material powder is not particularly limited, as long as it is a cosmetic material and may be made into powder. Examples of the cosmetic material powder may include inorganic powders, such as silicic acid, silicic anhydride, magnesium silicate, talc, kaoline, mica, sericite, bentonite, titanium coated mica, bismuth oxychloride, zirconium oxide, magnesium oxide, zinc oxide, titanium oxide, calcium carbonate, magnesium carbonate, iron oxide, ultramarine, prussian blue, chromium oxide, chromium hydroxide, calamine, and zeolite; and organic powders, suchascellulosepowder, silkpowder, nylonpowder, polyethylene powder, polystyrene powder, and polypropylene powder, as well as polyamide, polyester, polyethylene, polypropylene, polystyrene, polyurethane, vinyl resin, urea resin, phenol resin, fluorocarbon resin, silicon resin, acrylic resin, melamine resin, epoxy resin, polycarbonate resin, divinylbenzene-styrene resin, and cellulose resin.

The particle size of the cosmetic material powder may suitably be selected depending on the kind of the cosmetic product to be prepared, and may usually be about 10 nm to 100 µm.

The surface-treatment of the cosmetic material powder with the GU polymer may be performed by dissolving the GU polymer in a suitable solvent, soaking the cosmetic material powder in the solution, removing the solvent, and drying the powder.

In the solution of the GU polymer, the content of the GU polymer is not particularly limited, and may usually be about 0.01 to 10 wt%. At less than 0.01 wt%, uniform coating over the powder surface may be difficult, and at over 10 wt%, the powder may aggregate.

The cosmetic product according to the present invention contains the GU polymer and also a cosmetic material.

The cosmetic material may suitably be selected from conventional cosmetic materials, depending on the kind of the cosmetic product of the present invention to be produced. As a cosmetic material containing the GU polymer, the powder for cosmetics obtained by surface-treating the cosmetic material powder with the GU polymer may also be used.

At least a part of the cosmetic material contained in the cosmetic product may be in powder form, and this cosmetic material in powder form may be entirely or partially surface-treated with the GU polymer.

The cosmetic product according to the present invention may be in the form of, for example, cosmetics for basic skin care, such as lotion, emulsion, cream, and essence; make-up cosmetics, such as foundation, eye color, cheek color, and lip color; hair cosmetics, such as hair tonic, hair cream, and conditioner; cleansing cosmetics, such as shampoo and soap; nail cosmetics, such as nail color; and bath agents, such as bath bubbles.

Examples of the cosmetic material may include water, low molecular compounds having hydroxyl groups, such as ethanol, 1,3-butylene glycol, polyethylene glycol, glycerin, diglycerin, and polyglycerin; water-soluble polymers, such as sodium chondroitin sulfate, hyarulonic acid, arabic gum, sodium alginate, carrageenan, methylcellulose, hydroxyethylcellulose, carboxymethylcellulose, carboxyvinyl polymer, polyvinyl alcohol, polyvinylpyrrolidone, and sodium polyacrylate; surfactants, such as anionic, cationic, and amphoteric surfactants; and phosphatidylcholine, phosphatidylserine, phosphatidylethanolamine, phosphatidylglycerol, phosphatidylinositol, sphingophospholipid, silicone oil, oils and fats, hydrocarbons, higher fatty acid esters, amino acid derivatives, fluorochemical oil solutions, highly polymerized dimethylpolysiloxane, alkoxy-modified silicone, hydrocarbon wax, and lanolin derivatives. One or a mixture of two or more of these may be used.

In the cosmetic product of the present invention, the ratio of the GU polymer and the cosmetic material may suitably be selected for achieving desired effects depending on the kind or the like of the cosmetic product. It is preferred to decide the ratio so that the content of the GU polymer is usually 0.001 to 50 wt%, particularly 0.01 to 30 wt%.

The cosmetic product of the present invention may be produced in accordance with a conventional method, depending on the kinds of the cosmetic product or the cosmetic material.

Since the cosmetic product of the present invention contains the GU polymer, inherent functions of ceramide as an intercellular lipid, such as skin barrier function and hair protection effect, may be fully expressed, and a good texture is given to the cosmetic product. Thus the cosmetic product of the present invention protects skin against irritants due to is excellent barrier function, and when in the form of hair cosmetics or a cleansing agent, protects hair against irritants. Since the nanoparticles for cosmetics and the powder for cosmetics according to the present invention contain the ceramide-like GU polymer, excellent skin barrier function and hair protection effect are expressed, with ease of incorporation into various cosmetic materials.

The present invention will now be explained in more detail with reference to Examples, which are illustrative only and do not intend to limit the present invention.

### Synthesis Example 1: Synthesis of Glycerol-1-methacryloyloxyethyl Urethane

A four-neck flask was charged with 330 g of synthesized (R,S)-1,2-isopropylideneglycerol and 50 ml of pyridine, and equipped with a dropping funnel and a calcium tube. 368 g of methacryloyloxyethylisocyanate (manufactured by SHOWA DENKO K. K. ) was measured out, and slowly added dropwise into the flask at room temperature in dark. The resulting mixture was reacted for 7 hours in an oil bath at 50 °C. After the reaction was completed, pyridine and the excess (R,S)-1,2-isopropylideneglycerol were evaporated under reduced pressure, to thereby obtain 621 g of (R,S)-1,2-isopropylideneglycerol-3-methacryloyloxyethy 1 urethane in the form of white solid at 91% yield.

500 g of the (R,S)-1,2-isopropylideneglycerol-3-methacryloyloxyethy 1 urethane thus obtained was mixed with 1. 95 L of methanol and 50 ml of 4N hydrochloric acid, and reacted under stirring at roomtemperature for 30 minutes, which made the suspension into a clear solution. The solution was reacted under stirring for further 60 minutes, and the solvent was removed by drying under reduced pressure, to thereby obtain 412 g of glycerol-1-methacryloyloxyethyl urethane (abbreviated as GMU) in the form of a colorless viscous liquid at 96 % yield.

### Synthesis Example 2: Synthesis of GMU Homopolymer

20.0 g of GMU dissolved in 140 g of ethanol was placed in a four-neck flask, bubbled with nitrogen for 30 minutes, mixed with 0.12 g of 2,2'-azobisisobutyronitrile at 60 °C, and polymerized for 8 hours. The polymer liquid was added dropwise into 3 L of diethyl ether under stirring. The resulting precipitate was separated by filtration, and vacuum dried at room temperature for 48 hours, to thereby obtain 15.1 g of powder. This polymer powder is referred to as (P-1).

The molecular weight was analyzed by gel permeation chromatography (GPC), using methanol as an eluent and polyethylene glycol as a reference material. The detection was made by refractive index. The results of the analysis are shown in Table 1.

### Synthesis Examples 3 to 5: Synthesis of GMU Copolymer

GMU, butylmethacrylate (abbreviated as BMA), and stearylmethacrylate (abbreviated as SMA) were used as monomers for copolymerization, mixed in accordance with the monomer composition as shown in Table 1, and subjected to solution polymerization in the same way as in Synthesis Example 2. The resulting GMU polymers are referred to as (P-2) to (P-4), and the molecular weights were measured in the same way as in Synthesis Example 2. The results are shown in Table 1.

**Table 1**

| | Polymer powder | Mw | Monomer composition (mass%) | | |
|---|---|---|---|---|---|
| | | | GMU | BMA | SMA |
| Synthesis Example 2 | (P-1) | 72000 | 100 | - | - |
| Synthesis Example 3 | (P-2) | 52000 | 70 | 30 | - |
| Synthesis Example 4 | (P-3) | 28000 | 30 | 70 | - |
| Synthesis Example 5 | (P-4) | 32000 | 40 | - | 60 |

### Preparation Example 1: Preparation of Nanospheres

To 2.0g of (P-4) powder synthesized in Synthesis Example 5, 18.0g of a 1, 3-butanediol/glycerine mixed solution (5/5 by mass ratio) was added, and vigorously stirred in water bath at 70 °C, to obtain a turbid viscous liquid. 60 g of water at 70 °C was added little by little into this viscous liquid under stirring, to obtain a blue-white, scattering, nanosphere dispersion (referred to as (N-1)) at a concentration of 2.5 mass%. A portion of the dispersion was taken out and diluted with water to measure the particle size by dynamic light scattering using NICOMP 380ZLS (registered trade mark, manufactured by PARTICLE SIZING SYSTEMS). The particle size was found to be 25 nm. Further, the particle size was measured again four weeks after the production of (N-1) , and found to be 26 nm, which was constant. The dispersion remained stable without aggregation.

### Examples 1-1 to 1-5, Comparative Examples 1-1 and 1-2: Lotion

According to the prescription in Table 2, the components listed in row (a) were dissolved at room temperature. Separately, the components listed in row (b) were dissolved uniformly at 60 °C, and added into the solution of (a) under stirring, to thereby prepare a lotion. This lotion was subjected to the following sensory evaluation and safety test. The results are shown in Table 2.

### <Sensory Evaluation>

Ten women at 21 to 55 years of age were made to apply a suitable amount of the lotion on the inside of their forearm, and evaluated the spreadability, smoothness, and affinity to the skin in five grades according to the following levels. The evaluation points were averaged for scoring.

### Evaluation Points

5 points: very good; 4 points: good; 3 points: moderate; 2 points: slightly bad; 1 point: bad

### Score

average point of 4.0 or higher: (A); average point of not lower than 3.0 and lower than 4.0: (B); average point of not lower than 2.0 and lower than 3.0: (C); and average point of not lower than 1.0 and lower than 2.0: (D)

### <Safety Test>

50 ml of the lotion was placed in a sample bottle, capped, and kept still in a thermostatic chamber at 40 °C immediately after the preparation. The bottle was taken out one month later, and the state of the solution was visually observed and evaluated in three grades according to the following levels.

### Evaluation Code

### (A): no insolubles; (B): slight insolubles; (C) apparent insolubles

### <Test for Measuring Irritation Inhibitory Effect>

Ten ICR mice per group (male, 20 to 30 g of body weight) were used to determine the defensive effect of the lotion against irritation. Specifically, the mice were shaved on their back, 0.05 ml of the lotion was applied thereon, and the applied site was closed-patched with 0.05 ml of a 1 wt% aqueous solution of sodium lauryl sulfate for 24 hours. This operation was repeated four times, and 72 hours after the removal of the final patch, the scale, erythema, and conductance of the skin were measured. The scale and erythema were scored by assigning 2 points for the severely observed, 1 point for the apparently observed, and 0.5 points for the slightly observed, and the average was taken as the result. The conductance (µΩ⁻¹) was measured with a corneometer.

**Table 2**

| | | | Example | | | | | Comparative Example | |
|---|---|---|---|---|---|---|---|---|---|
| | | | 1-1 | 1-2 | 1-3 | 1-4 | 1-5 | 1-1 | 1-2 |
| | Ethanol | | 5.00 | 5.00 | 5.00 | 5.00 | 5.00 | 5.00 | 5.00 |
| (a) | Preservative | | proper amount | proper amount | proper amount | proper amount | proper amount | proper amount | proper amount |
| (b) | (P-1) | | 0.40 | - | - | - | - | - | - |
| | (P-2) | | - | 0.40 | - | - | - | - | - |
| | (P-3) | | - | - | 0.40 | - | - | - | - |
| | (P-4) | | - | - | - | 0.40 | - | - | - |
| | (N-1) | | - | - | - | - | 16.00 | - | - |
| | Ceramide | | - | - | - | - | - | 0.40 | - |
| | Glyoerin | | 1.00 | 1.00 | 1.00 | 1.00 | 1.00 | 1.00 | 1.00 |
| | Dipropylene glycol | | 5.00 | 5.00 | 5.00 | 5.00 | 5.00 | 5.00 | 5.00 |
| | Polyethylene glycol | | 2.00 | 2.00 | 2.00 | 2.00 | 2.00 | 2.00 | 2.00 |
| | Polyoxyethylene caster wax (60) | | 0.90 | 0.90 | 0.90 | 0.90 | 0.90 | 0.90 | 0.90 |
| | Tetrasodium EDTA | | 0.10 | 0.10 | 0.10 | 0.10 | 0.10 | 0.10 | 0.10 |
| | Sodium citrate | | 0.20 | 0.20 | 0.20 | 0.20 | 0.20 | 0.20 | 0.20 |
| | Ion-exchanged water | | balance | balance | balance | balance | balance | balance | balance |
| Evaluation | | Spreadability | (A) | (A) | (B) | (A) | (A) | (C) | (D) |
| | | Smoothness | (B) | (B) | (B) | (B) | (A) | (B) | (C) |
| | | Affinity to Skin | (B) | (B) | (B) | (A) | (A) | (C) | (C) |
| Safety Test | | | (B) | (B) | (B) | (C) | (B) | (D) | (A) |
| Irritation Inhibition | | Scale | 0.9 | 0.9 | 1.0 | 0.8 | 0.7 | 1.6 | 1.8 |
| | | Erythema | 0.8 | 0.9 | 1.0 | 0.7 | 0.6 | 1.5 | 1.8 |
| | | Conductance | 36 | 33 | 36 | 40 | 45 | 18 | 16 |

### Examples 2-1 to 2-5, Comparative Examples 2-1 and 2-2: Emulsion

According to the prescription in Table 3, the components listed in row (a) were uniformly dissolved at 75 °C. Separately, the components listed in row (b) were dissolved uniformly at 75 °C, to which the solution of (a) was added little by little to preemulsify. The mixture was then uniformly emulsified in a homomixer while the temperature was kept at 75 °C, and then cooled under stirring to give an emulsion. This emulsion was subjected to the sensory evaluation and safety test in the same way as in Examples 1-1 to 1-5. The results are shown in Table 3.

**Table 3**

| | | | Example | | | | | Comparative Example | |
|---|---|---|---|---|---|---|---|---|---|
| | | | 2-1 | 2-2 | 2-3 | 2-4 | 2-5 | 2-1 | 2-2 |
| (a) | White beeswax | | 2.50 | 2.50 | 2.50 | 2.50 | 2.50 | 2.50 | 2.50 |
| | Behenyl alcohol | | 1.00 | 1.00 | 1.00 | 1.00 | 1.00 | 1.00 | 1.00 |
| | Squalane | | 4.00 | 4.00 | 4.00 | 4.00 | 4.00 | 4.00 | 4.00 |
| | Stearic acid | | 1.00 | 1.00 | 1.00 | 1.00 | 1.00 | 1.00 | 1.00 |
| | Polyethylene glycol monostearate | | 2.50 | 2.50 | 2.50 | 2.50 | 2.50 | 2.50 | 2.50 |
| | Glycerin monostearate | | 0.50 | 0.50 | 0.50 | 0.50 | 0.50 | 0.50 | 0.50 |
| | Preservative | | proper amount | proper amount | proper amount | proper amount | proper amount | proper amount | proper amount |
| (b) | (P-1) | | 0.30 | - | - | - | - | - | - |
| | (P-2) | | - | 0.30 | - | - | - | - | - |
| | (P-3) | | - | - | 0.30 | - | - | - | - |
| | (P-4) | | - | - | - | 0.30 | - | - | - |
| | (N-1) | | - | - | - | - | 12.00 | - | - |
| | Ceramide | | - | - | - | - | - | 0.30 | - |
| | 1,3-butanediol | | 3.00 | 3.00 | 3.00 | 3.00 | 3.00 | 3.00 | 3.00 |
| | Dipropylene glycol | | 5.00 | 5.00 | 5.00 | 5.00 | 5.00 | 5.00 | 5.00 |
| | Potassium hydroxide | | 0.20 | 0.20 | 0.20 | 0.20 | 0.20 | 0.20 | 0.20 |
| | Tetrasodium EDTA | | 0.10 | 0.10 | 0.10 | 0.10 | 0.10 | 0.10 | 0.10 |
| | Sodium citrate | | 0.20 | 0.20 | 0.20 | 0.20 | 0.20 | 0.20 | 0.20 |
| | Ion-exchanged water | | balance | balance | balance | balance | balance | balance | balance |
| Evaluation | | Spreadability | (B) | (A) | (B) | (B) | (A) | (C) | (C) |
| | | Smoothness | (A) | (B) | (A) | (A) | (A) | (B) | (C) |
| | | Affinity to Skin | (B) | (B) | (B) | (A) | (A) | (C) | (C) |
| Safety Test | | | (B) | (B) | (B) | (C) | (B) | (D) | (B) |

### Examples 3-1 to 3-5, Comparative Examples 3-1 and 3-2: o/w Type Cream

According to the prescription in Table 4, the components listed in row (a) were uniformly dissolved at 75 °C. Separately, the components listed in row (b) were dissolved uniformly at 75 °C, to which the solution of (a) was added little by little to preemulsify. The mixture was then uniformly emulsified in a homomixer while the temperature was kept at 75 °C, and then cooled under stirring to give an o/w type cream. This cream was subjected to the sensory evaluation and safety test in the same way as in Examples 1-1 to 1-5. The results are shown in Table 4.

**Table 4**

| | | | Example | | | | | Comparative Example | |
|---|---|---|---|---|---|---|---|---|---|
| | | | 3-1 | 3-2 | 3-3 | 3-4 | 3-5 | 3-1 | 3-2 |
| (a) | Polyoxyethylene (20) | | 2.00 | 2.00 | 2.00 | 2.00 | 2.00 | 2.00 | 2.00 |
| | Sorbitan monooleate | | 5.00 | 5.00 | 5.00 | 5.00 | 5.00 | 5.00 | 5.00 |
| | Stearic acid | | 2.00 | 2.00 | 2.00 | 2.00 | 2.00 | 2.00 | 2.00 |
| | Cetanol | | 2.00 | 2.00 | 2.00 | 2.00 | 2.00 | 2.00 | 2.00 |
| | Squalane | | 16.00 | 16.00 | 16.00 | 16.00 | 16.00 | 16.00 | 16.00 |
| | Methylpolysiloxane | | 0.20 | 0.20 | 0.20 | 0.20 | 0.20 | 0.20 | 0.20 |
| | Preservative | | proper amount | proper amount | proper amount | proper amount | proper amount | proper amount | proper amount |
| (b) | (P-1) | | 0.50 | - | - | - | - | - | - |
| | (P-2) | | - | 0.50 | - | - | - | - | - |
| | (P-3) | | - | - | 0.50 | - | - | - | - |
| | (P-4) | | - | - | - | 0.50 | - | - | - |
| | (N-1) | | - | - | - | - | 20.00 | - | - |
| | Ceramide | | - | - | - | - | - | 0.50 | - |
| | 1,3-butanediol | | 7.00 | 7.00 | 7.00 | 7.00 | 7.00 | 7.00 | 7.00 |
| | Ion-exchanged water | | balance | balance | balance | balance | balance | balance | balance |
| Evaluation | | Spreadability | (B) | (B) | (B) | (B) | (A) | (C) | (C) |
| | | Smoothness | (A) | (A) | (B) | (A) | (A) | (C) | (C) |
| | | Affinity to Skin | (A) | (B) | (B) | (A) | (A) | (C) | (C) |
| Safety Test | | | (B) | (B) | (B) | (C) | (B) | (D) | (B) |

### Examples 4-1 to 4-5, Comparative Example 4-1 and 4-2: Hair Tonic

According to the prescription in Table 5, the components listed in row (a) were dissolved at room temperature. Separately, the components listed in row (b) were dissolved at 40 °C, to which the solution of (a) was added under stirring to give a hair tonic in the form of a lotion. The hair tonic thus obtained was subj ected to a sensory test conducted on ten males and females of 25 to 53 years of age, with respect to finger combability upon use, dry combability, and hair manageability. The hair tonic was also subjected to the safety test in the same way as in Examples 1-1 to 1-5. The evaluation and scoring in the sensory test was made in the same way as in Examples 1-1 to 1-5. The results are shown in Table 5.

**Table 5**

| | | | Example | | | | | Comparative Example | |
|---|---|---|---|---|---|---|---|---|---|
| | | | 4-1 | 4-2 | 4-3 | 4-4 | 4-5 | 4-1 | 4-2 |
| (a) | Ethanol | | 5.00 | 5.00 | 5.00 | 5.00 | 5.00 | 5.00 | 5.00 |
| | Methanol | | 1.00 | 1.00 | 1.00 | 1.00 | 1.00 | 1.00 | 1.00 |
| | Preservative | | proper amount | proper amount | proper amount | proper amount | proper amount | proper amount | proper amount |
| (b) | (P-1) | | 0.20 | - | - | - | - | - | - |
| | (P-2) | | - | 0.20 | - | - | - | - | - |
| | (P-3) | | - | - | 0.20 | - | - | - | - |
| | (P-4) | | - | - | - | 0.20 | - | - | - |
| | (N-1) | | - | - | - | - | 8.00 | - | - |
| | Ceramide | | - | - | - | - | - | 0.20 | - |
| | Swertia Japonica extract | | 0.01 | 0.01 | 0.01 | 0.01 | 0.01 | 0.01 | 0.01 |
| | Propylene glycol | | 5.00 | 5.00 | 5.00 | 5.00 | 5.00 | 5.00 | 5.00 |
| | Magnesium L-ascorbyl phosphate | | 1.00 | 1.00 | 1.00 | 1.00 | 1.00 | 1.00 | 1.00 |
| | Sodium edetate | | 0.03 | 0.03 | 0.03 | 0.03 | 0.03 | 0.03 | 0.03 |
| | Sodium citrate | | 0.70 | 0.70 | 0.70 | 0.70 | 0.70 | 0.70 | 0.70 |
| | Ion-exchanged water | | balance | balance | balance | balance | balance | balance | balance |
| Evaluation | | Finger capability upon use | (B) | (B) | (A) | (B) | (A) | (C) | (C) |
| | | Dry combability | (A) | (A) | (B) | (A) | (A) | (C) | (C) |
| | | Hair manageability | (B) | (B) | (B) | (A) | (A) | (C) | (C) |
| Safety Test | | | (B) | (B) | (B) | (C) | (B) | (D) | (B) |

### Examples 5-1 to 5-5, Comparative Examples 5-1 and 5-2: Shampoo

According to the prescription in Table 6, a shampoo was prepared. The obtained shampoo was subjected to the sensory evaluation in the same way as in Examples 4-1 to 4-5 and safety test in the same way as in Examples 1-1 to 1-5. The results are shown in Table 6.

**Table 6**

| | | Example | | | | | Comparative Example | |
|---|---|---|---|---|---|---|---|---|
| | | 5-1 | 5-2 | 5-3 | 5-4 | 5-5 | 5-1 | 5-2 |
| (P-1) | | 0.50 | - | - | - | - | - | - |
| (P-2) | | - | 0.50 | - | - | - | - | - |
| (P-3) | | - | - | 0.50 | - | - | - | - |
| (P-4) | | - | - | - | 0.50 | - | - | - |
| (N-1) | | - | - | - | - | 20.00 | - | - |
| Ceramide | | - | - | - | - | - | 0.50 | - |
| Polyoxyethylene (3 mol) sodium lauryl sulfate | | 10.0 | 10.0 | 10.0 | 10.0 | 10.0 | 10.0 | 10.0 |
| Lauryldimethylaminoacetic acid betaine | | 4.00 | 4.00 | 4.00 | 4.00 | 4.00 | 4.00 | 4.00 |
| | | | | | | | | |
| 1,3-butylene glycol | | 4.00 | 4.00 | 4.00 | 4.00 | 4.00 | 4.00 | 4.00 |
| Lauric diethanolamide | | 2.00 | 2.00 | 2.00 | 2.00 | 2.00 | 2.00 | 2.00 |
| Ethylene glycol distearate | | 2.00 | 2.00 | 2.00 | 2.00 | 2.00 | 2.00 | 2.00 |
| Cationic cellulose ¹⁾ | | 0.60 | 0.60 | 0.60 | 0.60 | 0.60 | 0.60 | 0.60 |
| Methylparaben | | 0.20 | 0.20 | 0.20 | 0.20 | 0.20 | 0.20 | 0.20 |
| Propylparaben | | 0.10 | 0.10 | 0.10 | 0.10 | 0.10 | 0.10 | 0.10 |
| Water | | balance | balance | balance | balance | balance | balance | balance |
| Evaluation | Finger combability upon use | (A) | (B) | (B) | (B) | (A) | (C) | (C) |
| | Dry combability | (A) | (B) | (B) | (A) | (A) | (C) | (C) |
| | Hair manageability | (B) | (A) | (B) | (A) | (A) | (B) | (C) |
| Safety test | | (B) | (B) | (B) | (C) | (B) | (D) | (B) |

| | | | | | | | | |
|---|---|---|---|---|---|---|---|---|
| 1) manufactured by UCC, trade name "polymer-JR-30M" | | | | | | | | |

### Examples 6-1 to 6-5, Comparative Examples 6-1 and 6-2: Conditioner

According to the prescription in Table 7, a conditioner was prepared. The obtained conditioner was subjected to the sensory evaluation in the same way as in Examples 4-1 to 4-5, and safety test in the same way as in Examples 1-1 to 1-5. The results are shown in Table 7.

**Table 7**

| | | Example | | | | | Comparative Example | |
|---|---|---|---|---|---|---|---|---|
| | | 6-1 | 6-2 | 6-3 | 6-4 | 6-5 | 6-1 | 6-2 |
| (P-1) | | 0.50 | - | - | - | - | - | - |
| (P-2) | | - | 0.50 | - | - | - | - | - |
| (P-3) | | - | - | 0.50 | - | - | - | - |
| (P-4) | | - | - | - | 0.50 | - | - | - |
| (N-1) | | - | - | - | - | 20.00 | - | - |
| Ceramide | | - | - | - | - | - | 0.50 | - |
| 1,3-butylene glycol | | 3.00 | 3.00 | 3.00 | 3.00 | 3.00 | 3.00 | 3.00 |
| Cetanol | | 2.00 | 2.00 | 2.00 | 2.00 | 2.00 | 2.00 | 2.00 |
| Glycerin monostearate | | 2.00 | 2.00 | 2.00 | 2.00 | 2.00 | 2.00 | 2.00 |
| Behenyltrimethylammonium chloride | | 2.00 | 2.00 | 2.00 | 2.00 | 2.00 | 2.00 | 2.00 |
| Octyldodecyl myristate | | 1.00 | 1.00 | 1.00 | 1.00 | 1.00 | 1.00 | 1.00 |
| Hydroxyethyl cellulose ¹⁾ | | 0.70 | 0.70 | 0.70 | 0.70 | 0.70 | 0.70 | 0.70 |
| Cationic cellulose ²⁾ | | 0.40 | 0.40 | 0.40 | 0.40 | 0.40 | 0.40 | 0.40 |
| Polyethylene glycol stearate (EO 5 mole) | | 0.30 | 0.30 | 0.30 | 0.30 | 0.30 | 0.30 | 0.30 |
| Polyethylene glycol stearate (EO 30 mole) | | 0.30 | 0.30 | 0.30 | 0.30 | 0.30 | 0.30 | 0.30 |
| Polyoxyethylene (20) sorbitan monostearate | | 0.30 | 0.30 | 0.30 | 0.30 | 0.30 | 0.30 | 0.30 |
| Methylparaben | | 0.20 | 0.20 | 0.20 | 0.20 | 0.20 | 0.20 | 0.20 |
| Propylparaben | | 0.10 | 0.10 | 0.10 | 0.10 | 0.10 | 0.10 | 0.10 |
| Water | | balance | balance | balance | balance | balance | balance | balance |
| Evaluation | Finger combability upon use | (B) | (A) | (A) | (B) | (A) | (C) | (C) |
| | Dry combability | (B) | (B) | (B) | (A) | (A) ) | (B) | (C) |
| | Hair manageability | (A) | (B) | (B) | (A) | (A) | (C) | (C) |
| Safety test | | (C) | (B) | (B) | (C) | (B) | (D) | (B) |

| | | | | | | | | |
|---|---|---|---|---|---|---|---|---|
| 1) manufactured by UCC, trade name "CEROSIZE QP-4400H" 2) manufactured by USS, trade name "POLYMER-JR-30M" | | | | | | | | |

### Examples 7-1 to 7-4, Comparative Examples 7-1: Foundation

30.0g of titanium oxide, 14.3 g of talc, 5.0g of mica titanium, 30.0 g of sericite, 5.0 g of fine titanium oxide, 5.0 g of fine zinc oxide, 8.0 g of yellow iron oxide, 2.0 g of red iron oxide, and 0.7 g of black iron oxide were measured out, placed in a Henschel mixer, and mixed at high speed for 2 minutes, to obtain 100 g of toned powder, which is referred to as (UP-1).

On the other hand, each of (P-1) to (P-4) prepared in Synthesis Examples 2 to 5, respectively, was dissolved separately in a solvent of 10 wt% ethanol/60 wt% n-hexane/30 wt% acetone at a concentration of 0.1 wt% to prepare each polymer solution. In 100 ml of each polymer solution, 100 g of the above toned powder was soaked, stirred, and separated by suction filtration to take out the powder. The solvent was removed under stirring in an oven at 80 °C, and the resulting product was pulverized in a pulverizer equipped with a 1 mm herringbone screen and mixed to obtain 100 g of surface-coated toned powder, which is referred to as (CP-1) to (CP-4) after the polymer (P-1) to (P-4) used therein.

Then, according to the prescription in Table 8, the components listed in rows (a) and (c) were separately mixed and dissolved under heating at 80 °C. (CP-1) to (CP-4) or (UP-1) listed in row (b) were added to the solution of (a) and mixed in a mixer, to which the solution of (c) was added little by little to emulsify, and cooled under stirring to obtain 100 g of a foundation.

Ten females of 21 to 55 years of age were made to apply each foundation on the face, and evaluated the four items, i.e., moist feel, appearance, fit, and long-lastingness. The evaluation and scoring were made in the same way as in Examples 1-1 to 1-5. The results are shown in Table 8.

**Table 8**

| | | Example | | | | Comp. |
|---|---|---|---|---|---|---|
| | | 7-1 | 7-2 | 7-3 | 7-4 | 7-1 |
| (a) | Olive oil | 1.50 | 1.50 | 1.50 | 1.50 | 1.50 |
| | Isotridecyl isononanoate | 9.00 | 9.00 | 9.00 | 9.00 | 9.00 |
| | Octadodecyl oleate | 0.50 | 0.50 | 0.50 | 0.50 | 0.50 |
| | Butylparaben | 0.10 | 0.10 | 0.10 | 0.10 | 0.10 |
| | Tocopherol | 0.02 | 0.02 | 0.02 | 0.02 | 0.02 |
| | Squalane | 3.00 | 3.00 | 3.00 | 3.00 | 3.00 |
| | POE (20) sorbitan monostearate | 2.00 | 2.00 | 2.00 | 2.00 | 2.00 |
| | Sorbitan monooleate | 0.50 | 0.50 | 0.50 | 0.50 | 0.50 |
| | Glyceryl monostearate | 2.50 | 2.50 | 2.50 | 2.50 | 2.50 |
| | Stearic acid | 2.00 | 2.00 | 2.00 | 2.00 | 2.00 |
| (b) | (CP-1) | 15.00 | - | - | - | - |
| | (CP-2) | - | 15.00 | - | - | - |
| | (CP-3) | - | - | 15.00 | - | - |
| | (CP-4) | - | - | - | 15.00 | - |
| | (UP-1) | - | - | - | - | 15.00 |
| (c) | Propylene glycol | 6.00 | 6.00 | 6.00 | 6.00 | 6.00 |
| | Sodium Laurate | 0.10 | 0.10 | 0.10 | 0.10 | 0.10 |
| | Triethanol amine | 0.70 | 0.70 | 0.70 | 0.710 | 0.70 |
| | Methylparaben | 0.30 | 0.30 | 0.30 | 0.30 | 0.30 |
| | Ion-exchanged water | balance | balance | balance | balance | balance |
| Evaluation | Moist Feel | (B) | (B) | (A) | (A) | (D) |
| | Appearance | (A) | (A) | (B) | (A) | (C) |
| | Fit | (A) | (B) | (B) | (A) | (C) |
| | Long-lastingness | (B) | (B) | (A) | (A) | (C) |

From Tables 2 to 4, it is observed that the cosmetic products of the present invention were superior in all of the evaluation items, i.e. spreadability, smoothness, and affinity to the skin, compared to the cosmetic products in which the GU polymer was replaced with ceramide, and the cosmetic products which do not contain the GU polymer, as in Comparative Examples. It is also demonstrated that the cosmetic products containing the dispersion of the GU polymer in nanoparticle form, had particularly excellent feel of use and good stability in the product.

With the result of measurements of the irritation inhibitory effect as shown in Table 2, it is demonstrated that the cosmetic products of the present invention have the effect of inhibiting external chemical irritation, i.e., excellent barrier function. This barrier function was particularly remarkable with the cosmetic products containing the dispersion of the GU polymer in nanoparticle form.

The same tendency was observed in the hair cosmetics shown in Tables 5 to 7 and the make-up cosmetics shown in Table 8.

## Claims

1. A cosmetic product comprising:
a cosmetic material, and
a polymer obtained by polymerization of a monomer material comprising a glycerol (meth)acrylate monomer and a monomer having a long-chain alkyl group,
wherein said glycerol (meth)acrylate monomer is represented by the formula (1): 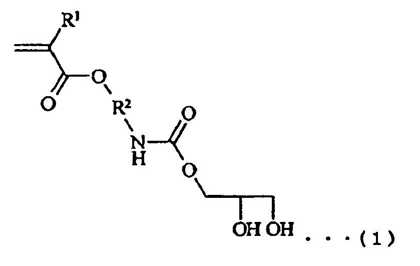 wherein R¹ stands for a hydrogen atom or a methyl group, and R² stands for -(CH₂)n- with n being an integer of 1 to 4, and
said monomer having a long-chain alkyl group is represented by the formula (2): 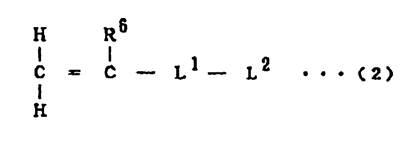 wherein L¹ stands for -C₆H₄, -C₆H₁₀-, -(C=O)-O-, -O-, -(C=O)NH-, -O-(C=O)-, or -O-(C=O)-O-, L² stands for an alkyl group having 10 to 22 carbon atoms, and R⁶ stands for a hydrogen atom or a methyl group.

2. The cosmetic product according to claim 1, wherein a content of said polymer is 0.001 to 50 wt% of the total weight of the cosmetic product.

3. The cosmetic product according to claim 1, wherein said polymer is in the form of nanoparticles having an average particle size of 5 to 500 nm.

4. The cosmetic product according to claim 1, wherein at least a part of said cosmetic material is in powder form, and said cosmetic material in powder form has been entirely or partially surface-treated with said polymer.

5. Use of a polymer obtained by polymerization of a monomer material comprising a glycerol (meth)acrylate monomer and a monomer having a long-chain alkyl group for the manufacture of a cosmetic product,
wherein said glycerol (meth)acrylate monomer is represented by the formula (1): 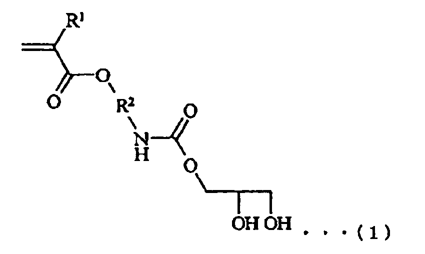 wherein R¹ stands for a hydrogen atom or a methyl group,
and R² stands for -(CH2)n- with n being an integer of 1 to 4, and
said monomer having a long-chain alkyl group is represented by the formula (2): 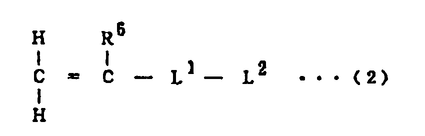 wherein L¹ stands for-C₆H₄-, -C₆H₁₀-, -(C=O) -O-, -O-, -(C=O)NH-, -O-(C=O)-, or -O-(C=O)-O-, L² stands for an alkyl group having 10 to 22 carbon atoms, and R⁶ stands for a hydrogen atom or a methyl group.

6. Nanoparticles for cosmetics comprising a polymer obtained by polymerization of a monomer material comprising a glycerol (meth)acrylate monomer represented by the formula (1): 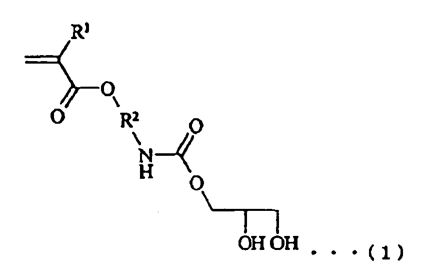 wherein R¹ stands for a hydrogen atom or a methyl group, and R² stands for - (CH₂)n- with n being an integer of 1 to 4, and
having an average particle size of 5 to 500 nm.

7. Powder for cosmetics comprising cosmetic material powder that has been surface-treated with a polymer obtained by polymerization of a monomer material comprising a glycerol (meth)acrylate monomer and a monomer having a long-chain alkyl group,
wherein said glycerol (meth)acrylate monomer is represented by the formula (1): 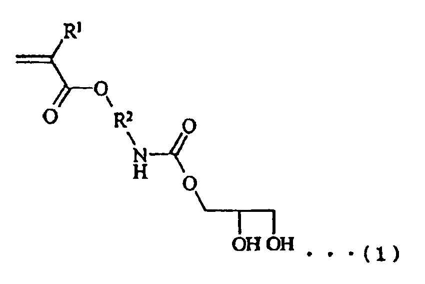 wherein R¹ stands for a hydrogen atom or a methyl group,
and R² stands for -(CH₂)n- with n being an integer of 1 to 4, and
said monomer having a long-chain alkyl group is represented by the formula (2): 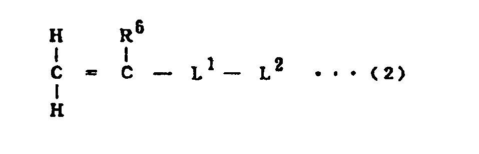 wherein L¹ stands for -C₆H₄-, -C₆H₁₀-, -(C=O)-O-, -O-, -(C=O)NH-, -O-(C=O)-, or -O-(C=O)-O-, L² stands for an alkyl group having 10 to 22 carbon atoms, and R⁶ stands for a hydrogen atom or a methyl group.

## Patentansprüche

1. Kosmetisches Produkt, umfassend:
ein kosmetisches Material und
ein Polymer, erhalten durch Polymerisation eines Monomer-Materials, das ein Glycerol(meth)acrylat-Monomer und ein Monomer mit einer langkettigen Alkylgruppe umfasst,
wobei das Glycerol(meth)acrylat-Monomer dargestellt wird durch die Formel (1): 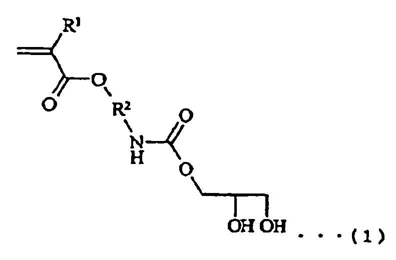 wobei R¹ für ein Wasserstoffatom oder eine Methylgruppe steht und R² für -(CH₂)ₙ-steht, wobei n eine ganze Zahl von 1 bis 4 ist, und
das Monomer mit einer langkettigen Alkylgruppe dargestellt wird durch die Formel (2): 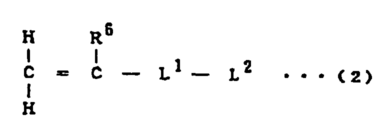 wobei L¹ für -C₆H₄-, -C₆H₁₀-, -(C=O)-O-, -O-, -(C=O)NH-, -O-(C=O)- oder -O-(C=O)-O- steht, L² für eine Alkylgruppe mit 10 bis 22 Kohlenstoffatomen steht und R⁶ für ein Wasserstoffatom oder eine Methylgruppe steht.

2. Kosmetisches Produkt nach Anspruch 1, wobei ein Gehalt des Polymers 0,001 bis 50 Gew.-% des Gesamtgewichtes des kosmetischen Produktes beträgt.

3. Kosmetisches Produkt nach Anspruch 1, wobei das Polymer in der Form von Nanoteilchen mit einer durchschnittlichen Teilchengröße von 5 bis 500 nm vorliegt.

4. Kosmetisches Produkt nach Anspruch 1, wobei wenigstens ein Teil des kosmetischen Materials in Pulverform vorliegt und das kosmetische Material in Pulverform vollständig oder teilweise mit dem Polymer oberflächenbehandelt worden ist.

5. Verwendung eines Polymers, erhalten durch Polymerisation eines Monomermaterials, das ein Glycerol(meth)acrylat-Monomer und ein Monomer mit einer langkettigen Alkylgruppe umfasst, zur Herstellung eines kosmetischen Produktes,
wobei das Glycerol(meth)acrylat-Monomer dargestellt wird durch die Formel (1): 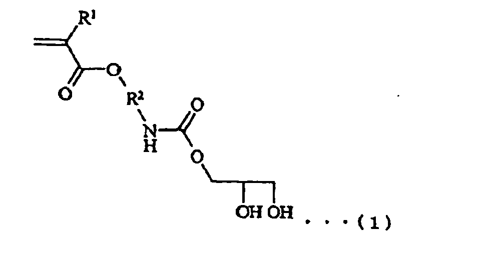 wobei R¹ für ein Wasserstoffatom oder eine Methylgruppe steht und R² für -(CH₂)ₙ-steht, wobei n eine ganze Zahl von 1 bis 4 ist, und
das Monomer mit einer langkettigen Alkylgruppe dargestellt wird durch die Formel (2): 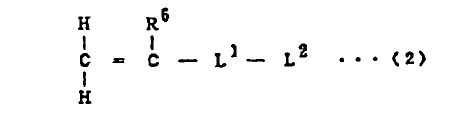 wobei L¹ für -C₆H₄- -C₆H₁₀-, -(C=O)-O-, -O-, -(C=O)NH-, -O-(C=O)- oder -O-(C=O)-O- steht, L² für eine Alkylgruppe mit 10 bis 22 Kohlenstoffatomen steht und R⁶ für ein Wasserstoffatom oder eine Methylgruppe steht.

6. Nanoteilchen für Kosmetika, umfassend ein Polymer, erhalten durch Polymerisation eines Monomermaterials, das ein Glycerol(meth)acrylat-Monomer umfasst, dargestellt durch die Formel (1): 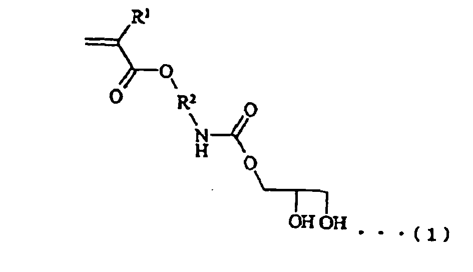 wobei R¹ für ein Wasserstoffatom oder eine Methylgruppe steht und R² für -(CH₂)ₙ-steht, wobei n eine ganze Zahl von 1 bis 4 ist, und
mit einer durchschnittlichen Teilchengröße von 5 bis 500 nm.

7. Pulver für Kosmetika, umfassend kosmetisches Materialpulver, das mit einem Polymer oberflächenbehandelt worden ist, erhalten durch Polymerisation eines Monomermaterials, das ein Glycerol(meth)acrylat-Monomer und ein Monomer mit einer langkettigen Alkylgruppe umfasst,
wobei das Glycerol(meth)acrylat-Monomer dargestellt wird durch die Formel (1): 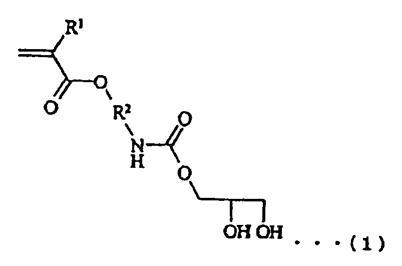 wobei R¹ für ein Wasserstoffatom oder eine Methylgruppe steht und R² für -(CH₂)ₙ-steht, wobei n eine ganze Zahl von 1 bis 4 ist, und
das Monomer mit einer langkettigen Alkylgruppe dargestellt wird durch die Formel (2): 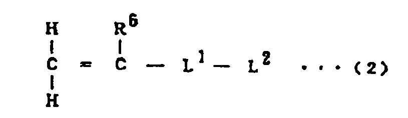 wobei L¹ für -C₆H₄-, -C₆H₁₀-, -(C=O)-O-, -O-, -(C=O)NH-, -O-(C=O)- oder -O-(C=O)-O- steht, L² für eine Alkylgruppe mit 10 bis 22 Kohlenstoffatomen steht und R⁶ für ein Wasserstoffatom oder eine Methylgruppe steht.

## Revendications

1. Produit cosmétique comprenant :
une matière cosmétique, et
un polymère obtenu par polymérisation d'une matière monomère comprenant un monomère de (méth)acrylate de glycérol et un monomère présentant un groupe alkyle à chaîne longue,
dans lequel ledit monomère de (méth)acrylate de glycérol est représenté par la formule (1) : 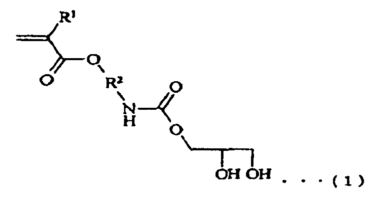 dans laquelle R¹ représente un atome d'hydrogène ou un groupe méthyle, et R² représente -(CH₂)n- avec n étant un nombre entier de 1 à 4, et
ledit monomère présentant un groupe alkyle à chaîne longue est représenté par la formule (2) : 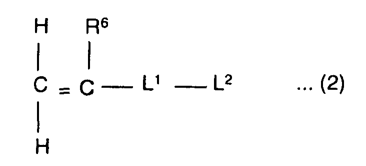
dans laquelle L¹ représente -C₆H₄, -C₆H₁₀-, -(C=O)-O-, -O-, -(C=O)NH-, -O-(C=O)-, ou -O-(C=O)-O-, L² représente un groupe alkyle ayant de 10 à 22 atomes de carbone, et R⁶ représente un atome d'hydrogène ou un groupe méthyle.

2. Produit cosmétique selon la revendication 1, dans lequel une teneur dudit polymère est de 0,001 à 50 % en poids du poids total du produit cosmétique.

3. Produit cosmétique selon la revendication 1, dans lequel ledit polymère est dans la forme de nanoparticules ayant une taille moyenne de particules de 5 à 500 nm.

4. Produit cosmétique selon la revendication 1, dans lequel au moins une partie de ladite matière cosmétique est dans une forme de poudre, et ladite matière cosmétique dans une forme de poudre a été entièrement ou partiellement traitée en surface avec ledit polymère.

5. Utilisation d'un polymère obtenu par polymérisation d'une matière monomère comprenant un monomère de (méth)acrylate de glycérol et un monomère présentant un groupe alkyle à chaîne longue pour la fabrication d'un produit cosmétique,
dans laquelle ledit monomère de (méth)acrylate de glycérol est représenté par la formule (1) : 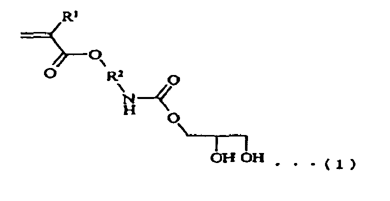 dans laquelle R¹ représente un atome d'hydrogène ou un groupe méthyle,
et R² représente -(CH₂)n- avec n étant un nombre entier de 1 à 4, et
ledit monomère présentant un groupe alkyle à chaîne longue est représenté par la formule (2) : 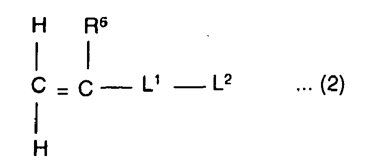 dans laquelle L¹ représente -C₆H₄-, -C₆H₁₀-, -(C=O)-O-, -O-, -(C=O)NH-, -O-(C=O)-, ou -O-(C=O)-O-, L² représente un groupe alkyle ayant de 10 à 22 atomes de carbone, et R⁶ représente un atome d'hydrogène ou un groupe méthyle.

6. Nanoparticules destinées à des cosmétiques comprenant un polymère obtenu par polymérisation d'une matière monomère comprenant
un monomère de (méth)acrylate de glycérol représenté par la formule (1) : 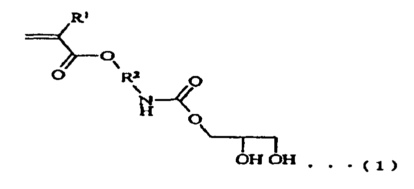 dans laquelle R¹ représente un atome d'hydrogène ou un groupe méthyle, et R² représente -(CH₂)n- avec n étant un nombre entier de 1 à 4, et
ayant une taille moyenne de particules de 5 à 500 nm.

7. Poudre pour cosmétiques comprenant une poudre de matière cosmétique qui a été traitée en surface avec un polymère obtenu par polymérisation d'une matière monomère comprenant un monomère de (méth)acrylate de glycérol et un monomère présentant un groupe alkyle à chaîne longue,
dans laquelle ledit monomère de (méth)acrylate de glycérol est représenté par la formule (1) : 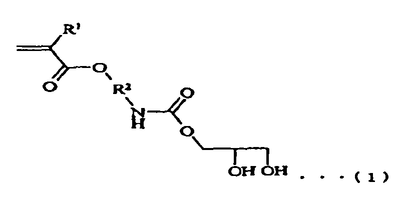 dans laquelle R¹ représente un atome d'hydrogène ou un groupe méthyle,
et R² représente -(CH₂)n- avec n étant un nombre entier de 1 à 4, et
ledit monomère présentant un groupe alkyle à chaîne longue est représenté par la formule (2) : 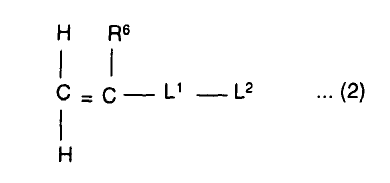 dans laquelle L¹ représente -C₆H₄-, -C₆H₁₀-, -(C=O)-O-, -O-, -(C=O)NH-, -O-(C=O)-, ou -O-(C=O)-O-, L² représente un groupe alkyle ayant de 10 à 22 atomes de carbone, et R⁶ représente un atome d'hydrogène ou un groupe méthyle.
